# EUROPÄISCHE PATENTSCHRIFT

(11) **EP 1 139 990 B1**
(45) Veröffentlichungstag und Bekanntmachung des Hinweises auf die Patenterteilung: **06.05.2004**
(21) Anmeldenummer: 99965463.5
(22) Anmeldetag: 14.12.1999
(51) Int. Cl.: A61K 7/13

(54) **MITTEL ZUM FÄRBEN VON KERATINHALTIGEN FASERN**
AGENT FOR DYING KERATIN FIBERS
AGENT PERMETTANT DE COLORER LES FIBRES DE KERATINE

(30) Priorität: 23.12.1998 DE 19859809
(43) Veröffentlichungstag der Anmeldung: 10.10.2001
(73) Patentinhaber: Henkel Kommanditgesellschaft auf Aktien, 40589 Düsseldorf-Holthausen (DE)
(72) Erfinder: MÖLLER, Hinrich, D-40789 Monheim (DE); OBERKOBUSCH, Doris, D-40591 Düsseldorf (DE); HÖFFKES, Horst, D-40595 Düsseldorf (DE)
(86) Internationale Anmeldenummer: PCT/EP1999/009911
(87) Internationale Veröffentlichungsnummer: WO 2000/038635

(56) Entgegenhaltungen:
- EP-A- 0 820 759
- DE-B- 2 932 489

## Beschreibung

Die Erfindung betrifft ein Mittel zum Färben von keratinhaltigen Fasern, insbesondere menschlichen Haaren, das aromatische Aldehyde oder Ketone mit quartären Ammoniumgruppen enthält, die Verwendung dieser Aldehyde oder Ketone als färbende Komponente in Haarfärbemitteln sowie ein Verfahren zum Färben von keratinhaltigen Fasern, insbesondere menschlichen Haaren.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsuntemehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch i.a. unter dem Einfluß von Oxidationsmitteln wie z. B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Die Verwendung der unten näher beschriebenen Aldehyde oder Ketone mit quartären Ammoniumgruppen zum Färben von keratinhaltigen Fasern ist bislang nicht bekannt.

Aufgabe der vorliegenden Erfindung ist es, Färbemittel für Keratinfasern, insbesondere menschliche Haare, bereitzustellen, die hinsichtlich der Farbtiefe, der Grauabdeckung und den Echtheitseigenschaften qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten Aldehyde oder Ketone mit quartären Ammoniumgruppen sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Der Einsatz von oxidierenden Agentien soll dabei jedoch nicht prinzipiell ausgeschlossen werden.

Gegenstand der Erfindung ist ein Mittel zum Färben von keratinhaltigen Fasern, insbesondere menschlichen Haaren, enthaltend aromatische Aldehyde oder Ketone mit quartären Ammoniumgruppen mit der Formel I, 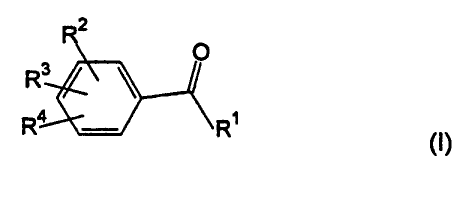 in der
- R¹: für Wasserstoff, eine C₁-C₄-Alkylgruppe oder eine gegebenenfalls substituierte Phenylgruppe steht,
- R² oder R³: gleich oder verschieden sind und für Wasserstoff, Halogen, Nitro, eine C₁-C₄-Alkylgruppe, eine C₁-C₄-Alkoxygruppe oder zusammen für einen ankondensierten aromatischen Ring stehen, oder
R¹ und R² zusammen mit der Carbonylfunktion für einen ankondensierten 5-oder 6-gliedrigen carbocyclischen Ring stehen,
R⁴ für eine Gruppe -N⁺R⁵R⁶R⁷X⁻ oder eine Gruppe mit der Formel II steht, 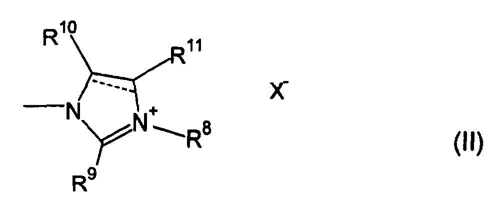 worin
R⁵ und R⁶ eine C₁-C₄-Alkylgruppe sind, die auch gemeinsam mit dem N-Atom einen 5-, 6- oder 7-gliedrigen Heterocyclus bilden können, und R⁵ auch zusammen mit R² oder R³ und dem aromatischen Ring einen benzokondensierten Heterocyclus bilden kann, der gegebenenfalls noch O, N oder S als weitere Heteroatome, Doppelbindungen, C₁-C₄-Alkyl- oder Arylsubstituenten oder einen weiteren ankondensierten aromatischen Ring aufweisen kann,
R⁷ und R⁸ unabhängig voneinander eine C₁-C₄-Alkylgruppe, C₁-C₄-Hydroxyalkylgruppe, eine gegebenenfalls substituierte Aryl-C₁-C₄-Alkylgruppe, eine Carboxy- oder Sulfo-(C₁-C₄)-alkylgruppe ist,
R⁹, R¹⁰ und R¹¹ gleich oder verschieden sein können und Wasserstoff, eine C₁-C₄-Alkylgruppe oder eine gegebenenfalls durch Halogen, C₁-C₄-Alkyl oder C₁-C₄-Alkoxy substituierte Phenylgruppe sind, wobei R¹⁰ und R¹¹ auch gemeinsam einen ankondensierten, gegebenenfalls substituierten aromatischen Ring bilden können,
X⁻ für Halogenid, ½ Sulfat, Hydrogensulfat, C₁-C₄-Alkylsulfat, C₁-C₄-Alkansulfonat, Trifluormethansulfonat, Arensulfonat, Perchlorhalogenat, Tetrafluorborat, ½ Tetrachlorzinkat, Fluorphosphat, Hydrogenphosphat, steht, und/oder 4-Trimethylammoniozimtaldehyd-methansulfonat.

Der zwei Stickstoffatome enthaltende 5-Ring in Formel (II) kann dabei für ein Imidazol- oder ein Imidazolin-System stehen.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose und synthetischer Fasern, wie z. B. Polyamid-, Polyacrylnitril-, Polyurethan- und Polyesterfasern verwendet werden.

Die Verbindungen mit der Formel I können auch in Form ihrer Hydrate eingesetzt werden.

Vorzugsweise sind die Verbindungen mit der Formel I ausgewählt aus der Gruppe folgender aromatischer Aldehyde und Ketone mit quartärer Ammoniumgruppe: 4-Trimethylammoniobenzaldehyd-methansulfonat, 4-Benzyldimethylammoniobenzaldhyd-bromid, 4-Trimethylammonionaphthaldehyd-methansulfonat, 2-Methoxy-4-trimethylammoniobenzaldehyd-methansulfonat, 4-Acetylphenyl-trimethylammonio-methansulfonat und -trifluormethansulfonat, 4-(N,N-Diethylmethylammonio)-benzaldehyd-methansulfonat, 4-Benzoylphenyl-trimethylammonium-iodid und -methansulfonat, N-(4-Benzoyl-phenyl)-N,N-diethylmethylammonium-iodid und - methansulfonat, N-(4-Formylphenyl)-N-methylpyrrolidinium-methansulfonat und - iodid, N-(4-Formylphenyl)-N-methylpiperidinium-methansulfonat, -methylsulfat und - p-toluolsulfonat, N-(4-Formylphenyl)-N-methylmorpholinium-methansulfonat, - methylsulfat und -p-toluolsulfonat, N-(4-Acetylphenyl)-N-methylmorpholiniummethansulfonat, -methylsulfat und -p-toluolsulfonat, N-(4-Benzoylphenyl)-N-methylmorpholinium-methansulfonat, - methylsulfat und -p-toluolsulfonat, 3-Formyl-N-ethyl-N-methylcarbazoliummethansulfonat, -methylsulfat und trifluormethansulfonat, 1-(4-Acetylphenyl)-3-methylimidazolium-iodid, -methansulfonat und -methylsulfat, 1-(4-Acetylphenyl)-3-methyl-2-imidazolinium-methansulfonat, -methylsulfat und -p-toluolsulfonat, 1-(4-Benzoylphenyl)-3-methylimidazolinium-methansulfonat und -methylsulfat, 5-Acetyl-1,3-diethyl-2-methylbenzimidazolium-iodid, 5-Trimethylammonio-1-indanoniodid und -methansulfonat, 3-Formyl-9-ethyl-9-methylcarbazolium-methansulfonat und -trifluormethansulfonat, 3-Formyl-9,9-dimethylcarbazolium-methansulfonat sowie deren beliebigen Gemische.

Diese Substanzen sind zum großen Teil literaturbekannt oder im Handel erhältlich oder nach bekannten Syntheseverfahren herstellbar.

Die voranstehend genannten aromatischen Aldehyde und Ketone mit quartärer Ammoniumgruppe mit der Formel I werden vorzugsweise in den erfindungsgemäßen Mitteln in einer Menge von 0,03 bis 65 mmol, insbesondere von 1 bis 40 mmol, bezogen auf 100 g des gesamten Färbemittels, verwendet. Sie können als direktziehende Färbemittel oder in Gegenwart von üblichen Oxidationsfarbstoffvorprodukten eingesetzt werden.

Färbemittel, die als färbende Komponente die aromatischen Aldehyde und Ketone mit quartärer Ammoniumgruppe mit der Formel I allein enthalten, werden bevorzugt für Färbungen im Gelb- und Orangebereich eingesetzt. Färbungen mit noch erhöhter Brillanz und verbesserten Echtheitseigenschaften (Lichtechtheit, Waschechtheit, Reibechtheit) über einen weiten Nuancenbereich von gelb über gelbbraun, orange, braunorange, mittelbraun, olivgrün, dunkelbraun, violett, dunkelviolett bis hin zu blauschwarz und schwarz werden erzielt, wenn die erfindungsgemäß eingesetzten Verbindungen mit der Formel I gemeinsam mit mindestens einer weiteren Komponente (im folgenden Komponente B genannt), ausgewählt aus Verbindungen mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder CH-aktiven Verbindungen, verwendet werden. Dies sind einerseits Verbindungen, die für sich alleine keratinhaltige Fasern nur schwach färben und erst gemeinsam mit den aromatischen Aldehyden und Ketonen der Formel I brillante Färbungen ergeben. Andererseits sind darunter aber auch Verbindungen, die bereits als Oxidationsfarbstoffvorprodukte eingesetzt werden.

Die voranstehend genannten Verbindungen der Komponente B können in einer Menge von jeweils 0,03 bis 65, insbesondere 1 bis 40 mmol, jeweils bezogen auf 100 g des gesamten Färbemittels, eingesetzt werden.

In allen Färbemitteln können auch mehrere verschiedene aromatische Aldehyde und Ketone der Formel I gemeinsam zum Einsatz kommen; ebenso können auch mehrere verschiedene Verbindungen der Komponente B gemeinsam verwendet werden.

Unter die voranstehend beschriebene Ausführungsform fällt auch die Verwendung von solchen Substanzen, die Reaktionsprodukte von aromatischen Aldehyden und Ketonen der Formel I mit den genannten Verbindungen der Komponente B darstellen, als direktziehende Färbemittel. Derartige Reaktionsprodukte können z. B. durch kurzes Erwärmen der beiden Komponenten in stöchiometrischen Mengen in wäßrigem neutralen bis schwach alkalischen Milieu erhalten werden, wobei sie entweder als Feststoff aus der Lösung ausfallen oder durch Eindampfen der Lösung daraus isoliert werden. Die Reaktionsprodukte können auch in Kombination mit anderen Farbstoffen oder Farbstoffvorprodukten eingesetzt werden.

Geeignete Verbindungen mit primärer oder sekundärer Aminogruppe als Komponente B sind z. B. primäre aromatische Amine wie N,N-Dimethyl-, N,N-Diethyl-, N-(2-Hydroxyethyl)-N-ethyl-, N,N-Bis-(2-hydroxyethyl)-, N-(2-Methoxyethyl-), 2,3-, 2,4-, 2,5-Dichlor-p-phenylendiamin, 2-Chlor-p-phenylendiamin, 2,5-Dihydroxy-4-morpholinoanilin-dihydrobromid, 2-, 3-, 4-Aminophenol, 2-Aminomethyl-4-aminophenol, 2-Hydroxymethyl-4-aminophenol, o-, p-Phenylendiamin, o-Toluylendiamin, 2,5-Diaminotoluol, -phenol, -phenethol, 4-Amino-3-methylphenol, 2-(2,5-Diaminophenyl)-ethanol, 2,4-Diaminophenoxyethanol, 2-(2,5-Diaminophenoxy)-ethanol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 6-Methyl-3-amino-2-chlor-, 2-Methyl-5-amino-4-chlor-, 3,4-Methylendioxy-, 5-(2-Hydroxyethylamino)-4-methoxy-2-methyl-, 4-Amino-2-hydroxymethylphenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxybenzoesäure, 2-, 3-, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 3,5-Diamino-4-hydroxybrenzcatechin, aromatische Nitrile, Aniline, insbesondere Nitrogruppen-haltige Aniline, wie 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest, wie sie in der Formel III dargestellt sind 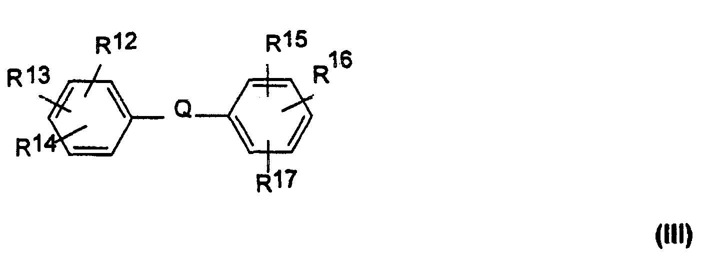 in der R¹² für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
R¹³, R¹⁴, R¹⁵, R¹⁶ und R¹⁷ für Wasserstoff, eine Hydroxy- oder eine Aminogruppe, die durch
C₁₋₄-Alkyl-, C₁₋₄-Hydroxyalkyl, C₁₋₄-Alkoxy-, C₁₋₄-Aminoalkyl- oder C₁₋₄-Alkoxy-C₁₋₄-alkylgruppen substituiert sein kann, stehen, und
Q für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel IV

**Z-(CH**_{**2**}**-Y-CH**_{**2**}**-Z')**_{**o**} **(IV)**

in der
Y eine direkte Bindung, eine CH₂- oder CHOH-Gruppe bedeutet,
Z und Z' unabhängig voneinander für ein Sauerstoffatom, eine NR¹⁸-Gruppe, worin R¹⁸ Wasserstoff, eine C₁₋₄-Alkyl- oder eine Hydroxy-C₁₋₄-alkylgruppe bedeutet, die Gruppe O-(CH₂)ₚ-NH oder NH-(CH₂)ₚ'-O, worin p und p' 2 oder 3 sind, stehen und
o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorind, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, -2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-, 3-, 4-Amino-, 2-Amino-3-hydroxy-, 2,6-Diamino-, 2,5-Diamino-, 2,3-Diamino-, 2-Dimethylamino-5-amino-, 2-Methylamino-3-amino-6-methoxy-, 2,3-Diamino-6-methoxy-, 2,6-Dimethoxy-3,5-diamino-, 2,4,5-Triamino-, 2,6-Dihydroxy-3,4-dimethylpyridin, 2,4-Dihydroxy-5,6-diamino-, 4,5,6-Triamino-, 4-Hydroxy-2,5,6-triamino-, 2-Hydroxy-4,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 3,5-Diaminopyrazol, -1,2,4-triazol, 3-Amino-, 3-Amino-5-hydroxypyrazol, 2-, 3-, 8-Aminochinolin, 4-Aminochinaldin, 2-, 6-Aminonicotinsäure, 5-Aminoisochinolin, 5-, 6-Aminoindazol, 5-, 7-Aminobenzimidazol, -benzothiazol, 2,5-Dihydroxy-4-morpholinoanilin sowie Indolund Indolinderivaten, wie 4-, 5-, 6-, 7-Aminoindol, 5,6-Dihydroxyindol, 5,6-Dihydroxyindolin und 4-Hydroxyindolin. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure.

Als CH-aktive Verbindungen können beispielhaft genannt werden 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Methyl-2-chinaldinium-iodid, 1-Ethyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

Als Aminosäuren kommen bevorzugt alle natürlich vorkommenden und synthetischen α-Aminosäuren in Frage, z. B. die durch Hydrolyse aus pflanzlichen oder tierischen Proteinen, z. B. Kollagen, Keratin, Casein, Elastin, Sojaprotein, Weizengluten oder Mandelprotein, zugänglichen Aminosäuren. Dabei können sowohl sauer als auch alkalisch reagierende Aminosäuren eingesetzt werden. Bevorzugte Aminosäuren sind Arginin, Histidin, Tyrosin, Phenylalanin, DOPA (Dihydroxyphenylalanin), Ornithin, Lysin und Tryptophan. Aber auch andere Aminosäuren, wie z. B. 6-Aminocapronsäure, können eingesetzt werden.

Die Oligopeptide können dabei natürlich vorkommende oder synthetische Oligopeptide, aber auch die in Polypeptid- oder Proteinhydrolysaten enthaltenen Oligopeptide sein, sofern sie über eine für die Anwendung in den erfindungsgemäßen Färbemitteln ausreichende Wasserlöslichkeit verfügen. Als Beispiele sind z. B. Glutathion und die in den Hydrolysaten von Kollagen, Keratin, Casein, Elastin, Sojaprotein, Weizengluten oder Mandelprotein enthaltenen Oligopeptide zu nennen. Bevorzugt sind dabei Mittel, die die Verbindungen mit der Formel I gemeinsam mit Verbindungen mit primärer oder sekundärer Aminogruppe oder mit aromatischen Hydroxyverbindungen enthalten.

Die Verbindungen der Komponente B werden besonders bevorzugt ausgewählt aus der Gruppe bestehend aus N-(2-Hydroxyethyl)-N-ethyl-, 2-Chlor-p-phenylendiamin, N,N-Bis(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie deren mit vorzugsweise anorganischen Säuren gebildeten physiologisch verträglichen Salze.

Auf die Anwesenheit von Oxidationsmitteln, z. B. H₂O₂, kann dabei verzichtet werden. Es kann jedoch u. U.. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

In einer bevorzugten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, z. B. aus der Gruppe der Nitrophenylendiamine, Nitroaminophenole, Anthrachinone oder Indophenole, wie z. B. die unter den internationalen Bezeichnungen bzw. Handelsnamen HC Yellow 2, HC Yellow 4, HC Yellow 6, Basic Yellow 57, Disperse Orange 3, HC Red 3, HC Red BN, Basic Red 76, HC Blue 2, Disperse Blue 3, Basic Blue 99, HC Violet 1, Disperse Violet 1, Disperse Violet 4, Disperse Black 9, Basic Brown 16 und Basic Brown 17 bekannten Verbindungen sowie Pikraminsäure 2-Amino-6-chloro-4-nitrophenol, 4-Amino-2-nitrodiphenylamin-2'-carbonsäure, 6-Nitro-1,2,3,4-tetrahydrochinoxalin, 4-N-Ethyl-1,4-bis(2'-hydroxyethylamino)-2-nitrobenzol-hydrochlorid und 1-Methyl-3-nitro-4-(2'-hydroxyethyl)-aminobenzol. Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie beispielsweise Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten.

Es ist nicht erforderlich, daß die Oxidationsfarbstoffvorprodukte oder die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder - SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2 Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxyl-amino-modifiziertes Silicon, das auch als Amodimethicone bebezeichnet wird), SM-2059 (Hersteller: General Electric), SLM-55067 (Hersteller: Wacker) sowie Abil®-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid®S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex® vertriebenen Methyl-hydroxyalkyl-di(alkanoyloxyalkyl)ammoniummethosulfate.

Ein Beispiel für ein als kationisches Tensid einsetzbares quaternäres Zuckerderivat stellt das Handelsprodukt Glucquat®100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copolymere, Vinylpyrrolidon-Imidazoliniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vemetzte Polyacrylsäuren, Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und Acrylsäure/Ethylacrylat/Ntert.-Butylacrylamid-Terpofymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammoniumoder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40, mmol bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 9.

Ein weiterer Gegenstand der vorliegenden Erfindung betrifft die Verwendung von aromatischen Aldehyden oder Ketonen mit quartären Ammoniumgruppen mit der Formel I, 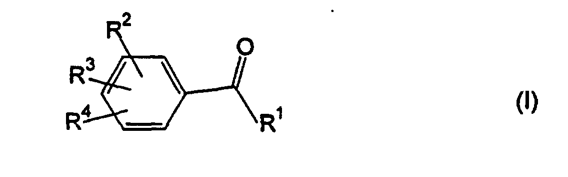 in der
R¹ für Wasserstoff, eine C₁-C₄-Alkylgruppe oder eine gegebenenfalls substituierte Phenylgruppe steht,
R² oder R³ gleich oder verschieden sein können und für Wasserstoff, Halogen, Nitro, eine C₁-C₄-Alkylgruppe, eine C₁-C₄-Alkoxygruppe oder zusammen für einen ankondensierten aromatischen Ring stehen, oder
R¹ und R² zusammen mit der Carbonylfunktion für einen ankondensierten 5- oder 6-gliedrigen carbocyclischen Ring stehen,
R⁴ für eine Gruppe -N⁺R⁵R⁶R⁷X⁻ oder eine Gruppe mit der Formel II steht, 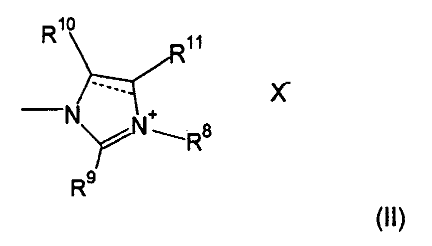 worin
R⁵ und R⁶ eine C₁-C₄-Alkylgruppe sind, die auch gemeinsam mit dem N-Atom einen 5-, 6- oder 7-gliedrigen Heterocyclus bilden können, und R⁵ auch zusammen mit R² oder R³ und dem aromatischen Ring einen benzokondensierten Heterocyclus bilden kann, der gegebenenfalls noch O, N oder S als weitere Heteroatome, Doppelbindungen, C₁-C₄-Alkyl- oder Arylsubstituenten oder einen weiteren ankondensierten aromatischen Ring aufweisen kann,
R⁷ und R⁸ unabhängig voneinander eine C₁-C₄-Alkylgruppe, C₁-C₄-Hydroxyalkylgruppe, eine gegebenenfalls substituierte Aryl-C₁-C₄-Alkylgruppe, eine Carboxy- oder Sulfo-(C₁-C₄)-alkylgruppe ist,
R⁹, R¹⁰ und R¹¹ gleich oder verschieden sein können und Wasserstoff, eine C₁-C₄-Alkylgruppe oder eine gegebenenfalls durch Halogen, C₁-C₄-Alkyl oder C₁-C₄-Alkoxy substituierte Phenylgruppe sind, wobei R¹⁰ und R¹¹ auch gemeinsam einen ankondensierten, gegebenenfalls substituierten aromatischen Ring bilden können,
X⁻ für Halogenid, ½ Sulfat, Hydrogensulfat, C₁-C₄-Alkylsulfat, C₁-C₄-Alkansulfonat, Trifluormethansulfonat, Arensulfonat, Perchlorhalogenat, Tetrafluorborat, ½ Tetrachlorzinkat, Fluorphosphat, Hydrogenphosphat, steht, und/oder 4-Trimethylammoniozimtaldehydmethansulfonat als eine färbende Komponente in Oxidationshaarfärbemitteln.

Noch ein weiterer Gegenstand der vorliegenden Erfindung betrifft ein Verfahren zum Färben von keratinhaltigen Fasern, insbesondere menschlichen Haaren, worin ein Färbemittel, enthaltend,
A) mindestens einen aromatischen Aldehyd oder ein aromatisches Keton mit quartären Ammoniumgruppen mit der Formel I, 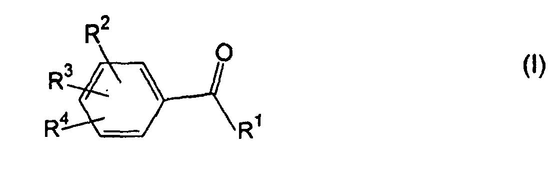 in der
   R¹ für Wasserstoff, eine C₁-C₄-Alkylgruppe oder eine gegebenenfalls substituierte Phenylgruppe steht,
   R² oder R³ gleich oder verschieden sein können und für Wasserstoff, Halogen, Nitro, eine C₁-C₄-Alkylgruppe, eine C₁-C₄-Alkoxygruppe oder zusammen für einen ankondensierten aromatischen Ring stehen, oder
   R¹ und R² zusammen mit der Carbonylfunktion für einen ankondensierten 5- oder 6-gliedrigen carbocyclischen Ring stehen,
   R⁴ für eine Gruppe -N⁺R⁵R⁶R⁷X⁻ oder eine Gruppe mit der Formel II steht, 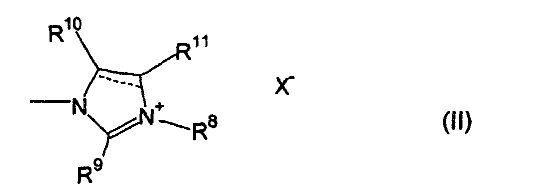 worin
   R⁵ und R⁶ eine C₁-C₄-Alkylgruppe sind, die auch gemeinsam mit dem N-Atom einen 5-, 6- oder 7-gliedrigen Heterocyclus bilden können, und R⁵ auch zusammen mit R² oder R³ und dem aromatischen Ring einen benzokondensierten Heterocyclus bilden kann, der gegebenenfalls noch O, N oder S als weitere Heteroatome, Doppelbindungen, C₁-C₄-Alkyl- oder Arylsubstituenten oder einen weiteren ankondensierten aromatischen Ring aufweisen kann,
   R⁷ und R⁸ unabhängig voneinander eine C₁-C₄-Alkylgruppe, C₁-C₄-Hydroxyalkylgruppe, eine gegebenenfalls substituierte Aryl-C₁-C₄-Alkylgruppe, eine Carboxy- oder Sulfo-(C₁-C₄)-alkylgruppe ist,
   R⁹, R¹⁰ und R¹¹ gleich oder verschieden sein können und Wasserstoff, eine C₁-C₄-Alkylgruppe oder eine gegebenenfalls durch Halogen, C₁-C₄-Alkyl oder C₁-C₄-Alkoxy substituierte Phenylgruppe sind, wobei R¹⁰ und R¹¹ auch gemeinsam einen ankondensierten, gegebenenfalls substituierten aromatischen Ring bilden können, X⁻ für Halogenid, ½ Sulfat, Hydrogensulfat, C₁-C₄-Alkylsulfat, C₁-C₄-Alkansulfonat, Trifluormethansulfonat, Arensulfonat, Perchlorhalogenat, Tetrafluorborat, ½ Tetrachlorzinkat, Fluorphosphat, Hydrogenphosphat, steht, und/oder 4-Trimethylammoniozimtaldehydmethansulfonat, und
B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

Die aromatischen Aldehyde oder Ketone der Formel I und die Verbindungen der Komponente B können entweder gleichzeitig auf das Haar aufgebracht werden oder aber auch nacheinander, wobei es unerheblich ist, welche der beiden Komponenten zuerst aufgetragen wird. Die fakultativ enthaltenen Ammonium- oder Metallsalze können dabei der ersten oder der zweiten Komponente zugesetzt werden. Zwischen dem Auftragen der ersten und der zweiten Komponente können bis zu 30 Minuten Zeitabstand liegen. Auch eine Vorbehandlung der Fasern mit der Salzlösung ist möglich.

Die aromatischen Aldehyde oder Ketone der Formel I und die Verbindungen der Komponente B können entweder getrennt oder zusammen gelagert werden, entweder in einer flüssigen bis pastösen Zubereitung (wäßrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30 bis 80°C) Wassers hinzugefügt und eine homogene Mischung hergestellt.

### Beispiele

### Herstellung einer Färbelösung

Es wurde je eine Aufschlämmung von 5 mmol eines aromatischen Aldehyds oder Ketons mit der Formel I, ggf. 5 mmol eines Oxidationsfarbstoffvorproduktes der Komponente B und 5 mmol Natriumacetat und einen Tropfen einer 20-%igen Fettalkylethersulfat-Lösung in 50 ml Wasser bei 60°C hergestellt. Die Aufschlämmungen wurden nach Abkühlen auf 30°C miteinander vermischt und mit verdünnter NaOH oder Salzsäure auf einen pH-Wert von 6 eingestellt.

In diese Färbelösung wurde bei 30°C 30 Minuten lang eine Strähne zu 90% ergrauten, nicht vorbehandelten Menschenhaares eingebracht. Die Strähne wurde dann 30 Sekunden mit lauwarmem Wasser gespült, mit warmer Luft getrocknet und anschließend ausgekämmt.

Die jeweiligen Farbnuancen und Farbtiefen sind in der nachfolgenden Tabelle 1 wiedergegeben.

Die Farbtiefe wurde dabei nach folgender Skala bewertet:
- - :: keine oder eine sehr blasse Ausfärbung
- (+) :: schwache Intensität
- + :: mittlere Intensität
- +(+) :: mittlere bis starke Intensität
- ++ :: starke Intensität
- ++(+) :: starke bis sehr starke Intensität
- +++ :: sehr starke Intensität

**Tabelle 1**

| Ausfärbungen mit 4-Trimethylammoniozimtaldehyd-methansulfonat | | |
|---|---|---|
| **Komponente B** | **Farbe** | **Intensität** |
| 2,5-Diaminotoluol x H₂SO₄ | dunkelrotviolett | +++ |
| 2-(2,5-Diaminophenyl)-ethanol x H₂SO₄ | dunkelbraunviolett | +++ |
| 2-Aminomethyl-4-aminophenol x 2 HCl | violettrot | ++(+) |

**Tabelle 2**

| Ausfärbungen mit 4-Trimethylammoniobenzaldehyd-methansulfonat | | |
|---|---|---|
| **Komponente B** | **Farbe** | **Intensität** |
| 2,5-Diaminotoluol x H₂SO₄ | orangebraun | ++ |
| 2,4,5,6-Tetraaminopyrimidin x H₂SO₄ | gelb | ++ |
| 1,8-Bis-(2,5-diaminophenoxy)-3,6-dioxaoctan x 4 HCl | dunkelbraun | ++(+) |
| 2-Methylamino-3-amino-6-methoxpyridin x 2 HCl | olivgrünorange | ++ |
| 2-(2,5-Diaminophenyl)-ethanol x H₂SO₄ | rotorange | ++ |
| 2-Aminomethyl-4-aminophenol x 2 HCl | goldgelb | ++ |
| N,N-Bis-(hydroxyethyl)-p-phenylendiamin x H₂SO₄ | violettrot | ++(+) |
| 4,4'-Diaminodiphenylamin x H₂SO₄ | schwarz | +++ |

**Tabelle 3**

| Ausfärbungen mit 4-Trimethylammonio-naphthaldehyd-trifuormethansulfonat | | |
|---|---|---|
| **Komponente B** | **Farbe** | **Intensität** |
| 2,5-Diaminotoluol x H₂SO₄ | mittelbraun | ++ |
| 2,4,5.6-Tetraaminopyrimidin x H₂SO₄ | gelborange-rot-braun | + |
| 1,8-Bis-(2,5-diaminophenoxy)-3,6-dioxaoctan x 4 HCl | schwarz | +++ |
| 2-(2,5-Diaminophenyl)-ethanol x H₂SO₄ | rotbraun | ++ |
| 2-Aminomethyl-4-aminophenol x 2 HCl | rotbraun | ++ |
| N,N-Bis-(hydroxyethyl)-p-phenylendiamin x H₂SO₄ | dunkelbraun | ++(+) |
| 4,4'-Diaminodiphenylamin x H₂SO₄ | schwarzbraun | +++ |
| 3,5-Diamino-2,6-dimethoxy-pyridin x 2 HCl | schwarz | +++ |

**Tabelle 4**

| Ausfärbungen mit 4-Trimethylammonio-acetophenon-trifuormethansulfonat | | |
|---|---|---|
| **Komponente B** | **Farbe** | **Intensität** |
| -- | - | - |
| 2,5-Diaminotoluol x H₂SO₄ | mittelbraun | +(+) |
| 2,4,5.6-Tetraaminopyrimidin x H₂SO₄ | gelborange | +(+) |
| 1,8-Bis-(2,5-diaminophenoxy)-3,6-dioxaoctan x 4 HCl | dunkelbraun | ++(+) |
| 2-Methylamino-3-amino-6-methoxy-pyridin x 2 HCl | graubraun | + |
| 2-(2,5-Diaminophenyl)-ethanol x H₂SO₄ | graubraun | + |
| 2-Aminomethyl-4-aminophenol x 2 HCl | gelbbraun | + |
| N,N-Bis-(hydroxyethyl)-p-phenylendiamin x H₂SO₄ | hellbraun | + |
| 4,4'-Diaminodiphenylamin x H₂SO₄ | braun | +++ |
| 3,5-Diamino-2,6-dimethoxy-pyridin x 2 HCl | schwarz | +++ |

**Tabelle 5**

| Ausfärbungen mit 1-(4-Acetylphenyl)-1-methyl-morpholinium-trifuormethansulfonat | | |
|---|---|---|
| **Komponente B** | **Farbe** | **Intensität** |
| -- | - | - |
| 2,5-Diaminotoluol x H₂SO₄ | graubraun | + |
| 2,4,5.6-Tetraaminopyrimidin x H₂SO₄ | braunorange | + |
| 1,8-Bis-(2,5-diaminophenoxy)-3,6-dioxaoctan x 4 HCl | schwarz | ++ |
| 2-Methylamino-3-amino-6-methoxy-pyridin x 2 HCl | dunkelbraun | ++(+) |
| 2-(2,5-Diaminophenyl)-ethanol x H₂SO₄ | graubraun | + |
| 2-Aminomethyl-4-aminophenol x 2 HCl | graubraun | + |
| N,N-Bis-(hydroxyethyl)-p-phenylendiamin x H₂SO₄ | hellbraun | +(+) |
| 4,4'-Diaminodiphenylamin x H₂SO₄ | schwarzgrün | +++ |
| 3,5-Diamino-2,6-dimethoxy-pyridin x 2 HCl | schwarz | +++ |

**Tabelle 6**

| Ausfärbungen mit 4-Trimethylammoniobenzophenon-methansulfonat | | |
|---|---|---|
| **Komponente B** | **Farbe** | **Intensität** |
| 2,5-Diaminotoluol x H₂SO₄ | mittelbraun | ++ |
| 2,4,5,6-Tetraaminopyrimidin x H₂SO₄ | goldgelb | ++ |
| 1,8-Bis-(2,4-diaminophenoxy)-3,6-dioxaoctan x 4 HCl | dunkelgrau | ++(+) |
| 2-Methylamino-3-amino-6-methoxypyridin x 2 HCl | dunkelbraun | ++(+) |
| 2-(2,5-Diaminophenyl)-ethanol x H₂SO₄ | hellbraun | + |
| 2-Aminomethyl-4-aminophenol x 2 HCl | hellbraun | + |
| N,N-Bis-(hydroxyethyl)-p-phenylendiamin x H₂SO₄ | hellbraun | + |
| 4,4'-Diaminodiphenylamin x H₂SO₄ | blauschwarz | +++ |
| 3,5-Diamino-2,6-dimethoxy-pyridin x 2 HCl* | schwarz | +++ |

**Tabelle 7**

| Ausfärbungen mit 1-(4-Acetylphenyl)-3-methylimidazolium-methansulfonat | | |
|---|---|---|
| **Komponente B** | **Farbe** | **Intensität** |
| 2,5-Diaminotoluol x H₂SO₄ | mittelbraun | +(+) |
| 2,4,5,6-Tetraaminopyrimidin x H₂SO₄ | braungelb | +(+) |
| 1,8-Bis-(2,5-diaminophenoxy)-3,6-dioxaoctan x 4 HCl | dunkelgrau | ++(+) |
| 2-Methylamino-3-amino-6-methoxypyridin x 2 HCl | dunkelbraun | ++(+) |
| 2-(2,5-Diaminophenyl)-ethanol x H₂SO₄ | graubraun | + |
| 2-Aminomethyl-4-aminophenol x 2 HCl | graubraun | + |
| N,N-Bis-(2-hydroxyethyl)-p-phenylendiamin x H₂SO₄ | graubraun | +(+) |
| 4,4'-Diaminodiphenylamin x H₂SO₄ | schwarz | +++ |
| 3,5-Diamino-2,6-dimethoxy-pyridin x 2HCl | schwarz | +++ |

## Patentansprüche

1. Mittel zum Färben von keratinhaltigen Fasern, insbesondere menschlichen Haaren, enthaltend als färbende Komponente mindestens einen aromatischen Aldehyd oder ein aromatisches Keton mit quartären Ammoniumgruppen mit der Formel I, 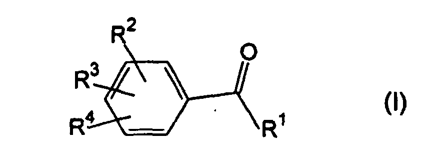 in der
R¹ für Wasserstoff, eine C₁-C₄-Alkylgruppe oder eine gegebenenfalls substituierte Phenylgruppe steht,
R² oder R³ gleich oder verschieden sein können und für Wasserstoff, Halogen, Nitro, eine C₁-C₄-Alkylgruppe, eine C₁-C₄-Alkoxygruppe oder zusammen für einen ankondensierten aromatischen Ring stehen, oder
R¹ und R² zusammen mit der Carbonylfunktion für einen ankondensierten 5- oder 6-gliedrigen carbocyclischen Ring stehen,
R⁴ für eine Gruppe -N⁺R⁵R⁶R⁷X⁻ oder eine Gruppe mit der Formel 11 steht, 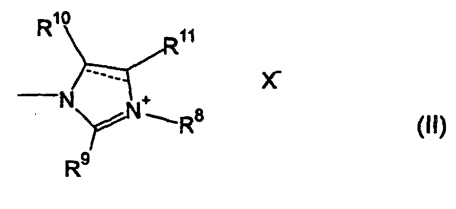 worin
R⁵ und R⁶ eine C₁-C₄-Alkylgruppe sind, die auch gemeinsam mit dem N-Atom einen 5-, 6- oder 7-gliedrigen Heterocyclus bilden können, und R⁵ auch zusammen mit R² oder R³ und dem aromatischen Ring einen benzokondensierten Heterocyclus bilden kann, der gegebenenfalls noch O, N oder S als weitere Heteroatome, Doppelbindungen, C₁-C₄-Alkyloder Arylsubstituenten oder einen weiteren ankondensierten aromatischen Ring aufweisen kann,
R⁷ und R⁸ unabhängig voneinander eine C₁-C₄-Alkylgruppe, C₁-C₄-Hydroxyalkylgruppe, eine gegebenenfalls substituierte Aryl-C₁-C₄-Alkylgruppe, eine Carboxy- oder Sulfo-(C₁-C₄)-alkylgruppe ist,
R⁹, R¹⁰ und R¹¹ gleich oder verschieden sein können und Wasserstoff, eine C₁-C₄-Alkylgruppe oder eine gegebenenfalls durch Halogen, C₁-C₄-Alkyl oder C₁-C₄-Alkoxy substituierte Phenylgruppe sind, wobei R¹⁰ und R¹¹ auch gemeinsam einen ankondensierten, gegebenenfalls substituierten aromatischen Ring bilden können,
X⁻ für Halogenid, ½ Sulfat, Hydrogensulfat, C₁-C₄-Alkylsulfat, C₁-C₄-Alkansulfonat, Trifluormethansulfonat, Arensulfonat, Perchlorhalogenat, Tetrafluorborat, ½ Tetrachlorzinkat, Fluorphosphat, Hydrogenphosphat, steht,
und/oder 4-Trimethylammoniozimtaldehyd.

2. Mittel nach Anspruch 1, **dadurch gekennzeichnet, daß** als Verbindungen mit der Formel I 4-Trimethylammoniobenzaldehyd-methansulfonat, 4-Benzyldimethylammoniobenzaldehyd-bromid, 4-Trimethylammonionaphthaldehyd-methansulfonat, 2-Methoxy-4-trimethylammoniobenzaldehyd-methansulfonat, 4-Acetylphenyl-trimethylammonio-methansulfonat und -trifluormethansulfonat, 4-(N,N-Diethylmethylammonio)-benzaldehyd-methansulfonat, 4-Benzoylphenyl-trimethylammonium-iodid und -methansulfonat, N-(4-Benzoylphenyl)-N,N-diethylmethylammonium-iodid und -methansulfonat, N-(4-Formylphenyl)-N-methylpyrrolidinium-methansulfonat und -iodid, N-(4-Formylphenyl)-N-methylpiperidinium-methansulfonat, -methylsulfat und -p-toluolfulfonat, N-(4-Formylphenyl)-N-methylmorpholinium-methansulfonat, -methylsulfat und -p-toluolfulfonat, N-(4-Acetylphenyl)-N-methylmorpholinium-methansulfonat, -methylsulfat und -p-toluolfulfonat, N-(4-Benzoylphenyl)-N-methylmorpholiniummethansulfonat, -methylsulfat und -p-toluolfulfonat, 3-Formyl-N-ethyl-N-methylcarbazolium-methansulfonat, -methylsulfat und trifluormethansulfonat, 1-(4-Acetylphenyl)-3-methylimidazolium-iodid, -methansulfonat und - methylsulfat, 1-(4-Acetylphenyl)-3-methyl-2-imidazolinium-methansulfonat, - methylsulfat und -p-toluolsulfonat, 1-(4-Benzoylphenyl)-3-methylimidazoliniummethansulfonat und -methylsulfat, 5-Acetyl-1,3-diethyl-2-methylbenzimidazolium-iodid, 5-Trimethylammonio-1-indanon-iodid und -methansulfonat, 3-Formyl-9-ethyl-9-methylcarbazolium-methansulfonat und - trifluormethansulfonat, 3-Formyl-9,9-dimethylcarbazolium-methansulfonat sowie deren beliebigen Gemische eingesetzt werden.

3. Mittel nach einem der Ansprüche 1 bis 2, **dadurch gekennzeichnet, daß** die aromatischen Aldehyde und Ketone mit der Formel I in einer Menge von 0,03 bis 65 mmol, insbesondere von 1 bis 40 mmol, bezogen auf 100 g des gesamten Färbemittels, enthalten sind.

4. Mittel nach einem der Ansprüche 1 bis 3, **dadurch gekennzeichnet, daß** es zusätzlich mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung, enthält.

5. Mittel nach Anspruch 4, **dadurch gekennzeichnet, daß** die weitere Verbindung ausgewählt ist aus primären oder sekundären Aminen aus der Gruppe, bestehend aus N-(2-Hydroxyethyl)-N-ethyl-, N-(2-Methoxyethyl-), 2,3-, 2,4-, 2,5-Dichlor-p-phenylendiamin, 2-Chlor-p-phenylendiamin, N,N-Bis-(2-hydroxyethyl)-p-phenylendiamin, 2,5-Dihydroxy-4-morpholinoanilin-dihydrobromid, 2-, 3-, 4-Aminophenol, o-, m-, p-Phenylendiamin, 2,4-Diaminophenoxyethanol, 2-(2,5-Diaminophenyl)-ethanol, 2,5-Diaminotoluol, -phenol, -phenethol, 4-Methylamino-, 3-Amino-4-(2'-hydroxyethyloxy)-, 3,4-Methylendiamino-, 3,4-Methylendioxyanilin, 3-Amino-2,4-dichlor-, 4-Methylamino-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 5-(2-Hydroxyethylamino)-4-methoxy-2-methyl-, 4-Amino-2-aminomethyl-phenol, 4-Amino-2-hydroxymethyl-phenol, 1,3-Diamino-2,4-dimethoxybenzol, 2-, 3-, 4-Aminobenzoesäure, -phenylessigsäure, 2,3-, 2,4-, 2,5-, 3,4-, 3,5-Diaminobenzoesäure, 4-, 5-Aminosalicylsäure, 3-Amino-4-hydroxy-, 4-Amino-3-hydroxy-benzoesäure, 2-, 3-, 4-Aminobenzolsulfonsäure, 3-Amino-4-hydroxybenzolsulfonsäure, 4-Amino-3-hydroxynaphthalin-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-, 1,2,4-Triaminobenzol, 1,2,4,5-Tetraaminobenzol-tetrahydrochlorid, 2,4,5-Triaminophenol-trihydrochlorid, Pentaaminobenzol-pentahydrochlorid, Hexaaminobenzol-hexahydrochlorid, 2,4,6-Triaminoresorcin-trihydrochlorid, 4,5-Diaminobrenzcatechinsulfat, 4,6-Diaminopyrogallol-dihydrochlorid, 3,5-Diamino-4-hydroxybrenzcatechin-sulfat, aromatische Nitrile, Aniline, insbesondere Nitrogruppen-haltige Aniline, wie 4-Nitroanilin, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 2-Amino-6-chlor-4-nitrophenol, 1-Amino-5-chlor-4-(2-hydroyethylamino)-2-nitrobenzol, aromatische Aniline bzw. Phenole mit einem weiteren aromatischen Rest wie 4,4'-Diaminostilben-dihydrochlorid, 4,4'-Diaminostilben-2,2'-disulfonsäure, Na-Salz, 4,4'-Diaminodiphenylmethan, -sulfid, -sulfoxid, -amin, 4,4'-Diaminodiphenylamin-2-sulfonsäure, 4,4'-Diaminobenzophenon, -diphenylether, 3,3',4,4'-Tetraamiriodiphenyl-tetrahydrochlorid, 3,3',4,4'-Tetraamino-benzophenon, 1,3-Bis-(2,4-diaminophenoxy)-propan-tetrahydrochlorid, 1,8-Bis-(2.5-diaminophenoxy)-3,6-dioxaoctan-tetrahydrochtorid, 1,3-Bis-(4-aminophenylamino)-propan, -2-propanol, 1,3-Bis-[N-(4-aminophenyl)-2-hydroxyethylamino]-2-propanol, N,N-Bis-[2-(4-aminophenoxy]-ethyl]methylamin-trihydrochlorid,
stickstoffhaltigen heterocyclischen Verbindungen ausgewählt aus der Gruppe, bestehend aus 2-, 3-, 4-Amino-, 2-Amino-3-hydroxy-, 2,6-Diamino-, 2,5-Diamino-, 2,3-Diamino-, 2-Dimethylamino-5-amino-, 3-Amino-2-methylamino-6-methoxy-, 2,3-Diamino-6-methoxy-, 3,5-Diamino-2,6-dimethoxy-, 2,4,5-Triamino-, 2,6-Dihydroxy-3,4-dimethylpyridin, 4,5,6-Triamino-, 2-Hydroxy-4,5,6-triamino-, 4-Hydroxy-2,5,6-triamino-, 2,4,5,6-Tetraamino-, 2-Methylamino-4,5,6-triamino-, 2,4-, 4,5-Diamino-, 2-Amino-4-methoxy-6-methyl-pyrimidin, 2,3,4-Trimethylpyrrol, 2,4-Dimethyl-3-ethyl-pyrrol, 3,5-Diaminopyrazol, -1,2,4-triazol, 3-Amino-, 3-Amino-5-hydroxypyrazol, 2-,3-, 8-Aminochinolin, 4-Aminochinaldin, 2-, 6-Aminonicotinsäure, 5-Aminoisochinolin, 5-, 6-Aminoindazol, 5-, 7-Amino-benzimidazol, -benzothiazol, 2,5-Dihydroxy-4-morpholinoanilin sowie Indol- und Indolinderivaten, wie 4-, 5-, 6-, 7-Aminoindol, 4-, 5-, 6-, 7-Hydroxyindol, 5,6-Dihydroxyindol, 5,6-Dihydroxyindolin und 4-Hydroxyindolin, sowie jeweils aus den mit vorzugsweise anorganischen Säuren gebildeten physiologisch verträglichen Salzen dieser Verbindungen,
aromatischen Hydroxyverbindungen ausgewählt aus der Gruppe, bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure, 3,6-Dihydroxy-2,7-naphthalinsulfonsäure, und
CH-aktiven Verbindungen ausgewählt aus der Gruppe, bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, Fischersche Base (1,3,3-Trimethyl-2-methylenindolin), 2,3-Dimethyl-benzothiazoliumiodid, 2,3-Dimethyl-benzothiazolium-p-toluolsulfonat, Rhodanin, Rhodanin-3-essigsäure, 1-Ethyl-2-chinaldinium-iodid, 1,4-Dimethylchinolinium-iodid, 1-Methyl-2-chinaldinium-iodid, 1,4-Dimethylchinolinium-iodid, Barbitursäure, Thiobarbitursäure, 1,3-Dimethylthiobarbitursäure, 1,3-Diethylthiobarbitursäure, Diethylthiobarbitursäure, Oxindol, 3-Indoxylacetat, Cumaranon und 1-Methyl-3-phenyl-2-pyrazolinon.

6. Mittel nach Anspruch 5, **dadurch gekennzeichnet, daß** die weitere Verbindung ausgewählt ist aus der Gruppe bestehend aus N-(2-Hydroxyethyl)-N-ethyl-, 2-Chlor-p-phenylendiamin, N,N-Bis(2-Hydroxyethyl)-p-phenylendiamin, 4-Aminophenol, p-Phenylendiamin, 2-(2,5-Diaminophenyl)-ethanol, 2,5-Diaminotoluol, 3,4-Methylendioxyanilin, 2-Amino-4-(2-hydroxyethylamino)-anisol, 2-(2,4-Diaminophenoxy)-ethanol, 3-Amino-2,4-dichlor-, 2-Methyl-5-amino-, 3-Methyl-4-amino-, 2-Methyl-5-(2-hydroxyethylamino)-, 2-Methyl-5-amino-4-chlor-, 6-Methyl-3-amino-2-chlor-, 2-Aminomethyl-4-aminophenol, 2-Hydroxymethyl-4-aminophenol, 3,4-Methylendioxyphenol, 3,4-Diaminobenzoesäure, 2,5-Diamino-, 2-Dimethylamino-5-amino-, 3-Amino-2-methylamino-6-methoxy-, 2,3-Diamino-6-methoxy-, 3,5-Diamino-2,6-dimethoxy-, 2,6-Dihydroxy-3,4-dimethylpyridin, 2-Hydroxy-4,5,6-triamino-, 4-Hydroxy-2,5,6-triamino-, 2,4,5,6-Tetraamino-, 2-Methylamino-4,5,6-triamino-pyrimidin, 3,5-Diaminopyrazol, 3-Amino-5-hydroxypyrazol, 5,6-Dihydroxyindol und 5,6-Dihydroxyindolin sowie jeweils aus den vorzugsweise mit anorganischen Säuren gebildeten physiologisch verträglichen Salzen dieser Verbindungen.

7. Mittel nach einem der Ansprüche 1 bis 6, **dadurch gekennzeichnet, daß** es direkt ziehende Farbstoffe aus der Gruppe der Nitrophenylendiamine, Nitroaminophenole, Anthrachinone oder Indophenole vorzugsweise in einer Menge von 0,01 bis 20 Gew.-%, bezogen auf das gesamte Färbemittel, enthält.

8. Mittel nach einem der Ansprüche 1 bis 7, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zuzugeben werden.

9. Mittel nach einem der Ansprüche 1 bis 8, **dadurch gekennzeichnet, daß** es anionische, zwitterionische oder nichtionische Tenside enthält.

10. Verwendung von aromatischen Aldehyden oder Ketonen mit quartären Ammoniumgruppen mit der Formel I gemäß Anspruch 1 und/oder von 4-Trimethylammoniozimtaldehyd als eine färbende Komponente in Oxidationshaarfärbemitteln.

11. Verfahren zum Färben von keratinhaltigen Fasern, insbesondere menschlichen Haaren, worin ein Färbemittel, enthaltend
A) mindestens einen aromatischen Aldehyd oder ein aromatisches Keton mit quartären Ammoniumgruppen mit der Formel I gemäß Anspruch 1 und/oder 4-Trimethylammoniozimtaldehyd,
B) mindestens eine Verbindung mit primärer oder sekundärer Aminogruppe oder Hydroxygruppe, ausgewählt aus primären oder sekundären aliphatischen oder aromatischen Aminen, stickstoffhaltigen heterocyclischen Verbindungen, Aminosäuren, aus 2 bis 9 Aminosäuren aufgebauten Oligopeptiden und aromatischen Hydroxyverbindungen, und/oder mindestens eine CH-aktive Verbindung,
sowie übliche kosmetische Inhaltsstoffe, auf die keratinhaltigen Fasern aufgebracht, einige Zeit, üblicherweise ca. 30 Minuten, auf der Faser belassen und anschließend wieder ausgespült oder mit einem Shampoo ausgewaschen wird.

12. Verfahren zum Färben von keratinhaltigen Fasern nach Anspruch 11, **dadurch gekennzeichnet, daß** das Färbemittel ein Oxidationsmittel in einer Menge von 0.01 bis 6 Gew.% bezogen auf die Anwendungslösung enthält.

## Claims

1. Preparations for colouring keratin-containing fibres, more particularly human hair, containing as colouring component at least one aromatic aldehyde or aromatic ketone containing quaternary ammonium groups corresponding to the following formula: 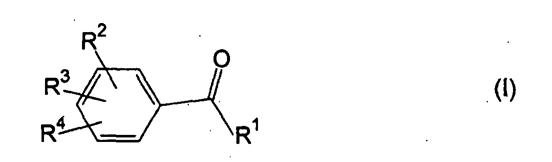 in which
R¹ is hydrogen, a C₁₋₄ alkyl group or an optionally substituted phenyl group,
R² and R³ may be the same or different and represent hydrogen, halogen, nitro, a C₁₋₄ alkyl group, a C₁₋₄ alkoxy group or, together, stand for a fused aromatic ring or
R¹ and R² together with the carbonyl function stand for a fused 5- or 6-membered carbocyclic ring,
R⁴ is a group -N⁺R⁵R⁶R⁷X⁻ or a group corresponding to formula II: 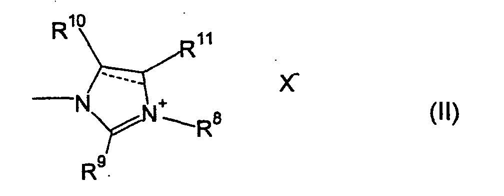 where R⁵ and R⁶ represent a C₁₋₄ alkyl group which, together with the N atom, may also form a 5-, 6- or 7-membered heterocycle and R⁵, together with R² or R³ and the aromatic ring, may also form a benzocondensed heterocycle which may optionally contain O, N or S as further hetero atoms, double bonds, C₁₋₄ alkyl or aryl substituents or another fused aromatic ring,
R⁷ and R⁸ independently of one another represent a C₁₋₄ alkyl group, a C₁₋₄ hydroxyalkyl group, an optionally substituted aryl-C₁₋₄-alkyl group, a carboxy or sulfo-(C₁₋₄)-alkyl group,
R⁹, R¹⁰ and R¹¹ may be the same or different and represent hydrogen, a C₁₋₄ alkyl group or an optionally halogen-, C₁₋₄-alkyl- or C₁₋₄-alkoxy-substituted phenyl group, in addition to which R¹⁰ and R¹¹ together may form a fused, optionally substituted aromatic ring,
X⁻ stands for halide, ½ sulfate, hydrogensulfate, C₁₋₄ alkyl sulfate, C₁₋₄ alkanesulfonate, trifluoromethanesulfonate, arene sulfonate, perchlorohalogenate, tetrafluroborate, ½ tetrachlorozincate, fluorophosphate, hydrogen phosphate,
and/or 4-trimethyl ammoniocinnamaldehyde.

2. A preparation as claimed in claim 1, **characterized in that** the compounds corresponding to formula I are 4-trimethyl ammoniobenzaldehyde methanesulfonate, 4-benzyldimethyl ammoniobenzaldehyde bromide, 4-trimethyl ammoniocinnamaldehyde methanesulfonate, 4-trimethyl ammonionaphthaldehyde methanesulfonate, 2-methoxy-4-trimethyl ammoniobenzaldehyde methanesulfonate, 4-acetylphenyl trimethyl ammoniomethanesulfonate and -trifluoromethanesulfonate, 4-(N,N-diethylmethylammonio)-benzaldehyde methanesulfonate, 4-benzoylphenyl trimethyl ammonium iodide and -methanesulfonate, N-(4-benzoylphenyl)-N,N-diethyl methyl ammonium iodide and -methanesulfonate, N-(4-formylphenyl)-N-methylpyrrolidinium methanesulfonate and -iodide, N-(4-formylphenyl)-N-methyl piperidinium methanesulfonate, -methyl sulfate and -p-toluene sulfonate, N-(4-formylphenyl)-N-methyl morpholinium methanesulfonate, -methyl sulfate and -p-toluenesulfonate, N-(4-acetylphenyl)-N-methyl morpholinium methanesulfonate, -methyl sulfate and -p-toluenesulfonate, N-(4-benzoylphenyl)-N-methyl morpholinium methanesulfonate, -methyl sulfate and -p-toluenesulfonate, 3-formyl-N-ethyl-N-methyl carbazolium methansulfonate, -methylsulfate and trifluoromethanesulfonate, 1-(4-acetylphenyl)-3-methyl imidazolium iodide, - methanesulfonate and -methyl sulfate, 1-(4-acetylphenyl)-3-methyl-2-imidazolinium methanesulfonate, -methyl sulfate and -p-toluenesulfonate, 1-(4-benzoylphenyl)-3-methylimidazolinium methanesulfonate and -methylsulfate, 5-acetyl-1,3-diethyl-2-methyl benzimidazolium iodide, 5-trimethylammonio-1-indanone iodide and -methanesulfonate, 3-formyl-9-ethyl-9-methylcarbazolium methanesulfonate and -trifluoromethanesulfonate, 3-formyl-9,9-dimethyl carbazolium methanesulfonate and mixtures thereof.

3. Preparations as claimed in claim 1 or 2, **characterized in that** the aromatic aldehydes and ketones corresponding to formula I are present in a quantity of 0.03 to 65 mmol and more particularly 1 to 40 mmol, based on 100 g of the colorant as a whole.

4. Preparations as claimed in any of claims 1 to 3, **characterized in that** they additionally contain at least one compound with a primary or secondary amino group or hydroxy group selected from primary or secondary aliphatic or aromatic amines, nitrogen-containing heterocyclic compounds, amino acids, oligopeptides of 2 to 9 amino acids and aromatic hydroxy compounds and/or at least one CH-active compound.

5. Preparations as claimed in claim 4, **characterized in that** the other compound is selected from primary or secondary amines from the group consisting of N-(2-hydroxyethyl)-N-ethyl-, N-(2-methoxyethyl-), 2,3-, 2,4-, 2,5-dichloro-p-phenylenediamine, 2-chloro-p-phenylenediamine, N,N-bis-(2-hydroxyethyl)-p-phenylenediamine, 2,5-dihydroxy-4-morpholinoaniline dihydrobromide, 2-, 3-, 4-aminophenol, o-, m-, p-phenylenediamine, 2,4-diaminophenoxyethanol, 2-(2,5-diaminophenyl)-ethanol, 2,5-diaminotoluene, -phenol, -phenethol, 4-methylamino-, 3-amino-4-(2'-hydroxyethyloxy)-, 3,4-methylenediamino-, 3,4-methylenedioxyaniline, 3-amino-2,4-dichloro-, 4-methylamino-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 5-(2-hydroxyethylamino)-4-methoxy-2-methyl-, 4-amino-2-aminomethyl phenol, 4-amino-2-hydroxymethyl phenol, 1,3-diamino-2,4-dimethoxybenzene, 2-, 3-, 4-aminobenzoic acid, -phenylacetic acid, 2,3-, 2,4-, 2,5-, 3,4-, 3,5-diaminobenzoic acid, 4-, 5-aminosalicylic acid, 3-amino-4-hydroxy-, 4-amino-3-hydroxybenzoic acid, 2-, 3-, 4-aminobenzenesulfonic acid, 3-amino-4-hydroxybenzenesulfonic acid, 4-amino-3-hydroxynaphthalenesulfonic acid, 6-amino-7-hydroxynaphthalene-2-sulfonic acid, 7-amino-4-hydroxynaphthalene-2-sulfonic acid, 4-amino-5-hydroxynaphthalene-2,7-disulfonic acid, 3-amino-2-naphthoic acid, 3-aminophthalic acid, 5-aminoisophthalic acid, 1,3,5-, 1,2,4-triaminobenzene, 1,2,4,5-tetraaminobenzene tetrahydrochloride, 2,4,5-triaminophenol trihydrochloride, pentaaminobenzene pentahydrochloride, hexaaminobenzene hexahydrochloride, 2,4,6-triaminoresorcinol trihydrochloride, 4,5-diaminopyrocatechol sulfate, 4,6-diaminopyrogallol dihydrochloride, 3,5-diamino-4-hydroxypyrocatechol sulfate, aromatic nitriles, anilines, more particularly anilines containing nitro groups, such as 4-nitroaniline, 4-nitro-1,3-phenylenediamine, 2-nitro-4-amino-1-(2-hydroxyethylamino)-benzene, 2-nitro-1-amino-4-[bis-(2-hydroxyethyl)-amino]-benzene, 4-amino-2-nitrodiphenylamine-2'-carboxylic acid, 2-amino-6-chlor-4-nitrophenol, 1-amino-5-chloro-4-(2-hydroxyethylamino)-2-nitrobenzene, aromatic anilines and phenols containing another aromatic groups, such as 4,4'-diaminostilbene dihydrochloride, 4,4'-diaminostilbene-2,2'-disulfonic acid, Na salt, 4,4'-diaminodiphenylmethane, -sulfide, -sulfoxide, -amine, 4,4'-diaminodiphenylamine-2-sulfonic acid, 4,4'-diaminobenzophenone, -diphenylether, 3,3',4,4'-tetraaminodiphenyl tetrahydrochloride, 3,3',4,4'-tetraaminobenzophenone, 1,3-bis-(2,4-diaminophenoxy)-propane tetrahydrochloride, 1,8-bis-(2,5-diaminophenoxy)-3,6-dioxaoctane tetrahydrochloride, 1,3-bis-(4-aminophenylamino)-propane, -2-propanol, 1,3-bis-[N-(4-aminophenyl)-2-hydroxyethylamino]-2-propanol, N,N-Bis-[2-(4-aminophenoxy)-ethyl]methylamine trihydrochloride,
nitrogen-containing heterocyclic compounds selected from the group consisting of 2-, 3-, 4-amino-, 2-amino-3-hydroxy-, 2,6-diamino-, 2,5-diamino-, 2,3-diamino-, 2-dimethylamino-5-amino-, 3-amino-2-methylamino-6-methoxy-, 2,3-diamino-6-methoxy-, 3,5-diamino-2,6-dimethoxy-, 2,4,5-triamino-, 2,6-dihydroxy-3,4-dimethylpyridine, 4,5,6-triamino-, 2-hydroxy-4,5,6-triamino-, 4-hydroxy-2,5,6-triamino-, 2,4,5,6-tetraamino-, 2-methylamino-4,5,6-triamino-, 2,4-, 4,5-diamino-, 2-amino-4-methoxy-6-methylpyrimidine, 2,3,4-trimethylpyrrole, 2,4-dimethyl-3-ethylpyrrole, 3,5-diaminopyrazole, - 1,2,4-triazole, 3-amino-, 3-amino-5-hydroxypyrazole, 2-,3-, 8-aminoquinoline, 4-aminoquinaldine, 2-, 6-aminonicotinic acid, 5-aminoisoquinoline, 5-, 6-aminoindazole, 5-, 7-aminobenzimidazole, -benzothiazole, 2,5-dihydroxy-4-morpholinoaniline and indole and indoline derivatives, such as 4-, 5-, 6-, 7-aminoindole, 4-, 5-, 6-, 7-hydroxyindole, 5,6-dihydroxyindole, 5,6-dihydroxyindoline and 4-hydroxyindoline and of the physiologically compatible salts of these compounds formed with preferably inorganic acids,
aromatic hydroxy compounds selected from the group consisting of 2-, 4-, 5-methylresorcinol, 2,5-dimethylresorcinol, resorcinol, 3-methoxyphenol, pyrocatechol, hydroquinone, pyrogallol, phloroglucinol, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulfonic acid, 3,6-dihydroxy-2,7-naphthalenesulfonic acid, and
CH-active compounds selected from the group consisting of 1,2,3,3-tetramethyl-3H-indolium iodide, 1,2,3,3-tetramethyl-3H-indolium-p-toluenesulfonate, 1,2,3,3-tetramethyl-3H-indolium methanesulfonate, Fischer's base (1,3,3-trimethyl-2-methyleneindoline), 2,3-dimethylbenzothiazolium iodide, 2,3-dimethyl benzothiazolium-p-toluenesulfonate, rhodanine, rhodanine-3-acetic acid, 1-ethyl-2-quinaldinium iodide, 1,4-dimethyl quinolinium iodide, 1-methyl-2-quinaldinium iodide, 1,4-dimethyl quinolinium iodide, barbituric acid, thiobarbituric acid, 1,3-dimethylthiobarbituric acid, 1,3-diethylthiobarbituric acid, diethylthiobarbituric acid, oxindole, 3-indoxylacetate, cuomaranone and 1-methyl-3-phenyl-2-pyrazolinone.

6. Preparations as claimed in claim 5, **characterized in that** the other compound is selected from the group consisting of N-(2-hydroxyethyl)-N-ethyl-, 2-chloro-p-phenylenediamine, N,N-bis(2-hydroacyethyl)-p-phenylenediamine, 4-aminophenol, p-phenylenediamine, 2-(2,5-diaminophenyl)-ethanol, 2,5-diaminotoluene, 3,4-methylenedioxyaniline, 2-amino-4-(2-hydroxyethylamino)-anisole, 2-(2,4-diaminophenoxy)-ethanol, 3-amino-2,4-dichloro-, 2-methyl-5-amino-, 3-methyl-4-amino-, 2-methyl-5-(2-hydroxyethylamino)-, 2-methyl-5-amino-4-chloro-, 6-methyl-3-amino-2-chloro-, 2-aminomethyl-4-aminophenol, 2-hydroxymethyl-4-aminophenol, 3,4-methylenedioxyphenol, 3,4-diaminobenzoic acid, 2,5-diamino-, 2-dimethylamino-5-amino-, 3-amino-2-methylamino-6-methoxy-, 2,3-diamino-6-methoxy-, 3,5-diamino-2,6-dimethoxy-, 2,6-dihydroxy-3,4-dimethylpyridine, 2-hydroxy-4,5,6-triamino-, 4-hydroxy-2,5,6-triamino-, 2,4,5,6-tetraamino-, 2-methylamino-4,5,6-triaminopyrimidine, 3,5-diaminopyrazole, 3-amino-5-hydroxypyrazole, 5,6-dihydroxyindole and 5,6-dihydroxyindoline and of the physiologically compatible salts of these compounds formed preferably with inorganic acids.

7. Preparations as claimed in any of claims 1 to 6, **characterized in that** they contain substantive dyes from the group of nitrophenylenediamines, nitroaminophenols, anthraquinones or indophenols preferably in a quantity of 0.01 to 20% by weight, based on the colorant as a whole.

8. Preparations as claimed in any of claims 1 to 7, **characterized in that** ammonium or metal salts selected from the group of formates, carbonates, halides, sulfates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium, or barium, or of aluminium, manganese, iron, cobalt, copper or zinc are added.

9. Preparations as claimed in any of claims 1 to 8, **characterized in that** they contain anionic, zwitterionic or nonionic surfactants.

10. The use of aromatic aldehydes or ketones containing quaternary ammonium groups corresponding to formula I in claim 1 and/or of 4-trimethyl ammoniocinnamaldehyde as a colouring component in oxidation hair colorants.

11. A process for colouring keratin-containing fibres, more particularly human hair, in which a colorant containing
A) at least one aromatic aldehyde or aromatic ketone containing quaternary ammonium groups corresponding to formula I in claim 1 and/or 4-trimethyl ammoniocinnamaldehyde,
B) at least one compound containing a primary or secondary amino group or hydroxy group selected from primary or secondary aliphatic or aromatic amines, nitrogen-containing heterocyclic compounds, amino acids, oligopeptides of 2 to 9 amino acids and aromatic hydroxy compounds, and/or at least one CH-active compound,
and typical cosmetic ingredients is applied to the keratin-containing fibres, left thereon for a time, usually ca. 30 minutes, and then rinsed out again or washed out with a shampoo.

12. A process for colouring keratin-containing fibres as claimed in claim 11, **characterized in that** the colorant contains an oxidizing agent in a quantity of 0.01 to 6% by weight, based on the solution applied.

## Revendications

1. Agent pour la teinture de fibres kératinisées, en particulier de cheveux humains, contenant, à titre de composant de coloration, au moins un aldéhyde aromatique ou une cétone aromatique comprenant des groupes d'ammonium quaternaires répondant à la formule I 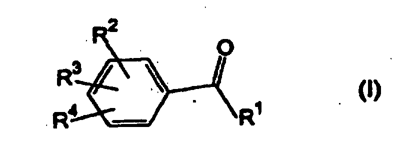 dans laquelle
R¹ représente un atome d'hydrogène, un groupe alkyle en C₁-C₄ ou un groupe phényle le cas échéant substitué ;
R² et R³ peuvent être identiques ou différents et représentent un atome d'hydrogène, un atome d'halogène, un groupe nitro, un groupe alkyle en C₁-C₄, un groupe alcoxy en C₁-C₄, ou représentent ensemble un noyau aromatique condensé ; ou bien
R¹ et R² représentent conjointement avec la fonction carbonyle, un noyau carbocyclique pentagonal ou hexagonal condensé ;
R⁴ représente un groupe -N⁺R⁵R⁶R⁷X⁻ ou un groupe répondant à la formule II 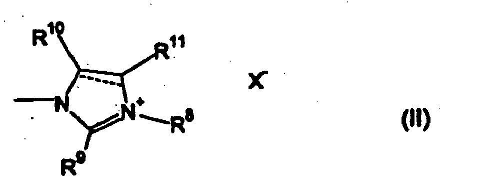 dans laquelle
R⁵ et R⁶ représentent un groupe alkyle en C₁-C₄, lesdits radicaux pouvant également former, ensemble avec l'atome d'azote, un groupe hétérocyclique pentagonal, hexagonal ou heptagonal, et R⁵ peut également former, conjointement avec R² ou R³ et avec le noyau aromatique, un composé hétérocyclique benzocondensé qui, le cas échéant, peut encore présenter un atome d'oxygène, un atome d'azote ou un atome de soufre, à titre d'hétéroatomes supplémentaires, des liaisons doubles, des substituants alkyle en C₁-C₄ ou aryle, ou encore un noyau aromatique condensé supplémentaire ;
R⁷ et R⁸ représentent, indépendamment l'un de l'autre, un groupe alkyle en C₁-C₄, un groupe hydroxyalkyle en C₁-C₄, un groupe arylalkyle en C₁-C₄ le cas échéant substitué, un groupe carboxy- ou sulfo-alkyle en C₁-C₄ ;
R⁹, R¹⁰ et R¹¹ peuvent être identiques ou différents et représentent un atome d'hydrogène, un groupe alkyle en C₁-C₄ ou un groupe phényle le cas échéant substitué par un atome d'halogène, par un groupe alkyle en C₁-C₄ ou par un groupe alcoxy en C₁-C₄, R¹⁰ et R¹¹ pouvant également former, ensemble, un noyau aromatique condensé, le cas échéant substitué ;
X⁻ représente un halogénure, un hémisulfate, un hydrogénosulfate, un alkyl(en C₁-C₄)sulfate, un alcane(en C₁-C₄)sulfonate, un trifluorométhanesulfonate, un arène-sulfonate, un perchlorohalogénate, un tétrafluoroborate, un hémitétrachlorozincate, un fluorophosphate, un hydrogénophosphate,
et/ou le 4-triméthylammoniocinnamaldéhyde.

2. Agent selon la revendication 1, **caractérisé en ce qu'**on met en oeuvre, à titre de composés répondant à la formule I, le méthanesulfonate du 4-triméthylammoniobenzaldéhyde, le bromure du 4-benzyldiméthylammoniobenzaldéhyde, le méthanesulfonate du 4-triméthylammonionaphtaldéhyde, le méthanesulfonate du 2-méthoxy-4-triméthylammonio-benzaldéhyde, le méthanesulfonate et le trifluorométhane-sulfonate du 4-acétylphényl-triméthylammonium, le méthanesulfonate du 4-(N,N-diéthylméthylammonio)-benzaldéhyde, l'iodure et le méthanesulfonate du 4-benzoyl-phényl-triméthylammonium, l'iodure et le méthanesulfonate du N-(4-benzoylphényl)-N,N-diéthylméthylammonium, le méthanesulfonate et l'iodure du N-(4-formylphényl)-N-méthyl pyrrolidinium, le méthanesulfonate, le méthylsulfate et le p-toluènesulfonate du N-(4-formylphényl)-N-méthylpipéridinium, le méthanesulfonate, le méthylsulfate et le p-toluènesulfonate du N-(4-formylphényl)-N-méthylmorpholinium, le méthane-sulfonate, le méthylsulfate et le p-toluènesulfonate du N-(4-acétylphényl)-N-méthylmorpholinium, le méthane-sulfonate, le méthylsulfate et le p-toluènesulfonate du N-(4-behzoylphényl)-N-méthylmorpholinium, le méthanesulfonate, le méthylsulfate et le trifluorométhanesulfonate du 3-formyl-N-éthyl-N-méthyl-carbazolium, l'iodure, le méthanesulfonate et le méthylsulfate du 1-(4-acétylphényl)-3-méthylimidazolium, le méthane-sulfonate, le méthylsulfate et le p-toluènesulfonate du 1-(4-acétylphényl)-3-méthyl-2-imidazolinium, le méthane-sulfonate et le méthylsulfate du 1-(4-benzoylphényl)-3-méthylimidazolinium, l'iodure du 5-acétyl-1,3-diéthyl-2-méthyl-2-benzimidazolium, l'iodure et le méthanesulfonate de la 5-triméthylammonio-1-indanone, le méthanesulfonate et le trifluorométhanesulfonate du 3-formyl-9-éthyl-9-méthyl-carbazolium, le méthanesulfonate du 3-formyl-9,9-diméthyl-carbazolium, ainsi que l'un quelconque de leurs mélanges.

3. Agent selon l'une quelconque des revendications 1 à 2, **caractérisé en ce qu'**il contient les cétones et les aldéhydes aromatiques répondant à la formule I en une quantité de 0,03 à 65 millimoles, en particulier de 1 à 40 millimoles, rapportés à 100 g de la totalité de la teinture.

4. Agent selon l'une quelconque des revendications 1 à 3, **caractérisé en ce qu'**il contient en outre, au moins un composé contenant un ou plusieurs groupes amino primaires ou secondaires ou un ou plusieurs groupes hydroxyle, choisis parmi le groupe comprenant des amines aromatiques ou aliphatiques primaires ou secondaires, des composés hétérocycliques azotés, des aminoacides, des oligopeptides constitués par 2 à 9 aminoacides et des composés hydroxylés aromatiques, et/ou au moins un composé CH-actif.

5. Agent selon la revendication 4, **caractérisé en ce que** le composé supplémentaire est choisi parmi
des amines primaires ou secondaires choisies parmi le groupe constitué par la N-(2-hydroxyéthyl)-N-éthyl-, la N-(2-méthoxyéthyl)-, la 2,3-, la 2,4-, la 2,5-dichloro-p-phénylènediamine, la 2-chloro-p-phénylènediamine, la N,N-bis-(2-hydroxyéthyl)-p-phénylène-diamine, le dibromhydrate de la 2,5-dihydroxy-4-morpholino-aniline, le 2-, le 3-, le 4-aminophénol, la o-, la m-, la p-phénylènediamine, le 2,4-diaminophénoxyéthanol, le 2-(2,5-diaminophényl)-éthanol, le 2,5-diamino-toluène, -phénol, -phénéthol, la 4-méthylamino-, la 3-amino-4-(2'-hydroxy-éthyloxy), la 3,4-méthylènediamino, la 3,4-méthylènedioxy-aniline, le 3-amino-2,4-dichloro-, le 4-méthylamino-, le 2-méthyl-5-amino-, le 3-méthyl-4-amino-, le 2-méthyl-5-(2-hydroxyéthylamino)-, le 2-méthyl-5-amino-4-chloro-, le 6-méthyl-3-amino-2-chloro-, le 5-(2-hydroxyéthylamino)-4-méthoxy-2-méthyl-, le 4-amino-2-aminométhyl-phénol, le 4-amino-2-hydroxyméthyl-phénol, le 1,3-diamino-2,4-diméthoxybenzène, l'acide 2-, l'acide 3-, l'acide 4-aminobenzoïque, l'acide 2-, l'acide 3-, l'acide 4-aminophénylacétique, l'acide 2,3-, l'acide 2,4-, l'acide 2,5-, l'acide 3,4-, l'acide 3,5-diaminobenzoïque, l'acide 4-, l'acide 5-aminosalicylique, l'acide 3-amino-4-hydroxy-, l'acide 4-amino-3-hydroxy-benzoïque, l'acide 2-, l'acide 3-, l'acide 4-aminobenzènesulfonique, l'acide 3-amino-4-hydroxybenzène-sulfonique, l'acide 4-amino-3-hydroxy-naphtalènesulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-amino-isophtalique, le 1,3,5-, le 1,2,4-triaminobenzène, le tétrachlorhydrate du 1,2,4,5-tétraaminobenzène, le trichlorhydrate du 2,4,5-triaminophénol, le pentachlorhydrate du pentaaminobenzène, l'hexachlorhydrate de l'hexaamino-benzène, le trichlorhydrate du 2,4,6-triaminorésorcinol, le sulfate du 4,5-diaminopyrocatéchol, le dichlorhydrate du 4,6-diaminopyrogallol, le sulfate du 3,5-diamino-4-hydroxy-pyrocatéchol, des nitriles aromatiques, des anilines, en particulier des anilines contenant un ou plusieurs groupes nitro, telles que la 4-nitroaniline, la 4-nitro-1,3-phénylènediamine, le 2-nitro-4-amino-1-(2-hydroxyéthylamino)-benzène, le 2-nitro-1-amino-4-[bis-(2-hydroxyéthyl)-amino]-benzène, l'acide 4-amino-2-nitrodiphénylamine-2'-carboxylique, le 2-amino-6-chloro-4-nitrophénol, le 1-amino-5-chloro-4-(2-hydroxyéthyl-amino)-2-nitrobenzène, des anilines aromatiques, respectivement des phénols aromatiques possédant un radical aromatique supplémentaire, tels que le dichlorhydrate du 4,4'-diamino-stilbène, l'acide 4,4'-diaminostilbène-2,2'-disulfonique, sel de sodium, le 4,4'-diaminodiphényl-méthane, -sulfure, -sulfoxyde, -amine, l'acide 4,4'-diaminodiphényl-amine-2-sulfonique, la 4,4'-diamino-benzophénone, l'éther 4,4'-diaminodiphénylique, le tétrachlorhydrate du 3,3',4,4'-tétraaminodiphényle, la 3,3',4,4'-tétraaminobenzophénone, le tétrachlorhydrate du 1,3-bis-(2,4-diaminophénoxy)-propane, le tétrachlorhydrate du 1,8-bis-(2,5-diaminophénoxy)-3,6-dioxa-octane, le 1,3-bis-(4-aminophénylamino)-propane, -2-propanol, le 1,3-bis-[N-(4-aminophényl)-2-hydroxyéthylamino]-2-propanol, le trichlorhydrate de la N,N-bis-[2-(4-amino phénoxy)-éthyl]-méthylamine,
des composés hétérocycliques azotés choisis parmi le groupe constitué par la 2-, la 3-, la 4-amino-, la 2-amino-3-hydroxy-, la 2,6-diamino-, la 2,5-diamino-, la 2,3-diamino-, la 2-diméthylamino-5-amino-, la 3-amino-2-méthylamino-6-méthoxy-, la 2,3-diamino-6-méthoxy-, la 3,5-diamino-2,6-diméthoxy-, la 2,4,5-triamino-, la 2,6-dihydroxy-3,4-diméthyl-pyridine, la 4,5,6-triamino-, la 2-hydroxy-4,5,6-triamino-, la 4-hydroxy-2,5,6-triamino-, la 2,4,5,6-tétraamino-, la 2-méthylamino-4,5,6-triamino-, la 2,4-, la 4,5-diamino-, la 2-amino-4-méthoxy-6-méthyl-pyrimidine, le 2,3,4-triméthylpyrrole, le 2,4-diméthyl-3-éthyl-pyrrole, le 3,5-diamino-pyrazole, - 1,2,4-triazole, le 3-amino-, le 3-amino-5-hydroxypyrazole, la 2-, la 3-, la 8-aminoquinoléine, la 4-amino-quinaldine, l'acide 2-, l'acide 6-aminonicotinique, la 5-amino-isoquinoléine, le 5-, le 6-amino-indazole, le 5-, le 7-amino-benzimidazole, -benzothiazole, la 2,5-dihydroxy-4-morpholino-aniline, ainsi que des dérivés d'indole et d'indoline, tels que le 4-, le 5-, le 6-, le 7-amino-indole, le 4-, le 5-, 6-, le 7-hydroxy-indole, le 5,6-dihydroxyindole, la 5,6-dihydroxyindoline et la 4-hydroxyindoline, ainsi que, respectivement, par les sels physiologiquement acceptables de ces composés, formés avec de préférence des acides inorganiques,
des composés hydroxylés aromatiques choisis parmi le groupe constitué par le 2-, le 4-, le 5-méthylrésorcinol, le 2,5-diméthyl résorcinol, le résorcinol, le 3-méthoxyphénol, le pyrocatéchol, l'hydroquinone, le pyrogallol, la phloroglucine, l'hydroxy-hydroquinone, le 2-, le 3-, le 4-méthoxy-, le 3-diméthylamino-, le 2-(2-hydroxyéthyl)-, le 3,4-méthylènedioxy-phénol, l'acide 2,4-, l'acide 3,4-dihydroxybenzoïque, - phénylacétique, l'acide gallique, l'acide 2,4,6-trihydroxybenzoïque, la 2,4,6-trihydroxy-acétophénone, le 2-, le 4-chlororésorcinol, le 1-naphtol, le 1,5-, le 2,3-, le 2,7-dihydroxynaphtalène, l'acide 6-diméthylamino-4-hydroxy-2-naphtalènesulfonique, l'acide 3,6-dihydroxy-2,7-naphtalènesulfonique, et des composés CH-actifs choisis parmi le groupe constitué par l'iodure du 1,2,3,3-tétraméthyl-3H-indolium, le p-toluène-sulfonate du 1,2,3,3-tétraméthyl-3H-indolium, le méthane-sulfonate du 1,2,3,3-tétraméthyl-3H-indolium, la base de Fischer (1,3,3-triméthyl-2-méthylène-indoline), l'iodure du 2,3-diméthyl-benzothiazolium, le p-toluènesulfonate du 2,3-diméthyl-benzothiazolium, la rhodanine, l'acide rhodanine-3-acétique, l'iodure du 1-éthyl-2-quinaldinium, l'iodure du 1,4-diméthylquinolinium, l'iodure du 1-méthyl-2-quinaldinium, l'acide barbiturique, l'acide thiobarbiturique, l'acide 1,3-diméthylthiobarbiturique, l'acide 1,3-diéthylthiobarbiturique, l'acide diéthylthiobarbiturique, l'oxoindole, le 3-indoxylacétate, la coumarannone et la 1-méthyl-3-phényl-2-pyrazolinone.

6. Agent selon la revendication 5, **caractérisé en ce que** le composé supplémentaire est choisi parmi le groupe constitué par la N-(2-hydroxyéthyl)-N-éthyl-, la 2-chloro-p-phénylène-diamine, la N,N-bis-(2-hydroxyéthyl)-p-phénylènediamine, le 4-aminophénol, la p-phénylènediamine, le 2-(2,5-diamino-phényl)-éthanol, le 2,5-diaminotoluène, la 3,4-méthylènedioxy-aniline, le 2-amino-4-(2-hydroxyéthylamino)-anisol, le 2-(2,4-diaminophénoxy)-éthanol, le 3-amino-2,4-dichloro-, le 2-méthyl-5-amino-, le 3-méthyl-4-amino-, le 2-méthyl-5-(2-hydroxyéthylamino)-, le 2-méthyl-5-amino-4-chloro-, le 6-méthyl-3-amino-2-chloro-, le 2-amino méthyl-4-aminophénol, le 2-hydroxyméthyl-4-aminophénol, le 3,4-méthylènedioxy-phénol, l'acide 3,4-diaminobenzoïque, la 2,5-diamino-, la 2-diméthylamino-5-amino-, la 3-amino-2-méthylamino-6-méthoxy-, la 2,3-diamino-6-méthoxy-, la 3,5-diamino-2,6-diméthoxy-, la 2,6-dihydroxy-3,4-diméthylpyridine, la 2-hydroxy-4,5,6-triamino-, la 4-hydroxy-2,5,6-triamino-, la 2,4,5,6-tétraamino-, la 2-méthylamino-4,5,6-triamino-pyrimidine, le 3,5-diaminopyrazole, le 3-amino-5-hydroxy-pyrazole, le 5,6-dihydroxyindole et la 5,6-dihydroxyindoline, ainsi que, respectivement, par les sels physiologiquement acceptables de ces composés, formés avec de préférence des acides inorganiques.

7. Agent selon l'une quelconque des revendications 1 à 6, **caractérisé en ce qu'**il contient des colorants montant directement sur la fibre, choisis parmi le groupe comprenant des nitrophénylènediamines, des nitroaminophénols, des anthraquinones ou des indophénols, de préférence en une quantité de 0,01 à 20 % en poids, rapportés à la totalité de la teinture.

8. Agent selon l'une quelconque des revendications 1 à 7, **caractérisé en ce qu'**on ajoute des sels d'ammonium ou des sels métalliques choisis parmi le groupe des formiates, des carbonates, des halogénures, des sulfates, des butyrates, des valérates, des caproates, des acétates, des lactates, des glycollates, des tartrates, des citrates, des gluconates, des propionates, des phosphates et des phosphonates de métaux alcalins tels que le potassium, le sodium ou le lithium, de métaux alcalino-terreux tels que le magnésium, le calcium, le strontium ou le baryum, ou encore de l'aluminium, du manganèse, du fer, du cobalt, du cuivre ou du zinc.

9. Agent selon l'une quelconque des revendications 1 à 8, **caractérisé en ce qu'**il contient des agents tensioactifs anioniques, amphotères ou non ioniques.

10. Utilisation d'aldéhydes ou de cétones aromatiques contenant des groupes d'ammonium quaternaires répondant à la formule I selon la revendication 1 et/ou du 4-triméthylammonio-cinnamaldéhyde, à titre de composant de coloration dans des teintures capillaires par oxydation.

11. Procédé pour la teinture de fibres kératinisées, en particulier de cheveux humains, dans lequel on applique, sur les fibres kératinisées, une teinture contenant
A) au moins un aldéhyde aromatique ou une cétone aromatique contenant des groupes d'ammonium quaternaires répondant à la formule I selon la revendication 1 et/ou du 4-triméthylammonio-cinnamaldéhyde,
B) au moins un composé contenant des groupes hydroxyle ou des groupes amino primaires ou secondaires, choisi parmi des amines aliphatiques ou aromatiques primaires ou secondaires, des composés hétérocycliques azotés, des aminoacides, des oligopeptides constitués par 2 à 9 aminoacides et des composés hydroxylés aromatiques, et/ou au moins un composé CH-actif,
ainsi que des constituants cosmétiques habituels, on la laisse agir sur les fibres pendant un certain temps, habituellement pendant environ 30 minutes, avant de l'éliminer par rinçage ou par lavage avec un shampooing.

12. Procédé pour la coloration de fibres kératinisées selon la revendication 11, **caractérisé en ce que** la teinture contient un agent d'oxydation en une quantité de 0,01 à 6 % en poids, rapportés à la solution d'application.
